# EUROPEAN PATENT SPECIFICATION

(11) **EP 3 866 885 B1**
(45) Date of publication and mention of the grant of the patent: **17.12.2025**
(21) Application number: 19872879.2
(22) Date of filing: 16.10.2019
(51) Int. Cl.: A61M 5/158, A61M 25/06, A61M 5/32, A61M 39/02, A61M 5/46, A61M 5/42

(54) **INSERTION DEVICE**
EINSETZVORRICHTUNG
DISPOSITIF D'INSERTION

(30) Priority: 16.10.2018 US 201862746351 P
(43) Date of publication of application: 25.08.2021
(73) Proprietor: Bio Health Frontiers, Inc., El Dorado Hills, CA 95765 (US)
(72) Inventor: LAMBERT, Paul, El Dorado Hills, California 95765 (US); GUTIERREZ, Carlos, El Dorado Hills, California 95762 (US)
(74) Representative: Jones Day
(86) International application number: PCT/US2019/056570
(87) International publication number: WO 2020/081708

(56) References cited:
- WO-A1-2009/007287
- US-A1- 2003 158 520
- US-A1- 2003 158 520
- US-A1- 2012 303 043
- US-A1- 2014 088 509
- US-A1- 2014 348 703
- US-A1- 2015 238 705
- US-A1- 2017 014 570

## Description

### FIELD

The disclosure relates generally to medical infusion systems. The disclosure relates specifically to insertion of a needle at a selected site within the body of a subject for subcutaneous, intravenous, intramuscular, or intradermal delivery of a drug to the subject.

### BACKGROUND

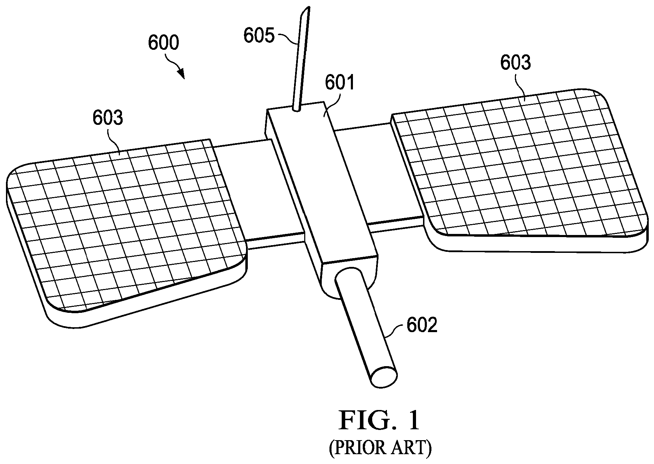

Medical needles are widely used in the course of patient treatment, particularly for delivery of selected medications. In one common form, hollow hypodermic needles are employed for transcutaneous delivery of a selected medication from a syringe or the like. In another common form, insertion needles are employed for transcutaneous placement of a soft and relatively flexible tubular cannula, followed by insertion needle removal and subsequent infusion of medical fluid to the patient through the cannula. Referring to Fig.1, a needle assembly 600 contains a main body 601, a pair of stabilization wings 603 attached to the main body 601, a medication supply tubing 602 is embedded into the main body 601 and communicates with a needle 605. Of course, a needle assembly 600 may be of simple or basic sort that includes only a needle 605 and, optionally, a main body 601 (whether or not the main body is aligned longitudinally with the needle 605) and/or medication supply tubing 602.

Certain therapies such as immunoglobulin therapy can be self-administered by a patient in the comfort of the patient's home. Infusion therapies require the user to insert a needle into the patient's body. While some patients have no difficulty self-inserting needles or receiving needles in their body, other patients are sensitive to the pain of the injection or are uncomfortable seeing needles or injecting needles into their body. In particular, many children have difficulty receiving infusion therapy due to the pain of needle insertion or fear of needles.

The method in which the needle is injected into the tissue is relevant to preventing pain. Research has shown that the speed of the insertion of the needle is important to protect the tissue layers from rupturing and reduce the pain of the insertion. When a needle is slowly inserted into the tissue, more rupture events are observed in the tissue as compared to the rapid insertion of a needle into tissue.

U.S. Pat. No. 6,830,562 describes an injection device to facilitate delivery of a dose of medicament to a patient through a hypodermic needle. The injection device can be administered by the patient themselves and can provide both psychological and physical advantages to patients. The device comprises a housing, a coil spring, and a safety device to offer a solution for automatic injection of medication. However, the apparatus is relatively complicated to manufacture and further the device has to be loaded manually by the patient by a rather complicated procedure. Further examples of prior art device are disclosed in US2003/158520A1, US2015/238705A1 and US2014/088509A1.

The present invention relates to an automatic insertion device, particularly for use with a subcutaneous infusion set, for quickly and easily placing an insertion needle and related cannula through the skin of a patient at the correct insertion angle, and with a speed and force of insertion which minimizes patient discomfort.

### SUMMARY

An object of the invention is to provide an improved insertion device which is easy to be manufactured and which is easily used by a patient.

In accordance with the invention, an insertion device is provided for quick and easy transcutaneous placement of a medical needle through the skin of a patient, particularly such as an insertion needle and related cannula of a subcutaneous infusion set. The insertion device is designed to place the needle through the skin at a selected place and with a controlled force and speed of insertion to ensure proper needle placement with minimal patient discomfort.

In one aspect, an insertion device comprises a cylindrical housing with a shell with a distal opening and a proximal opening; a carriage slidably received within the shell between a distal position and a retracted position; wherein said carriage has elements to secure a needle assembly.

The insertion device may include a drive for biasing the cylindrical housing relative to the carriage. In one embodiment, the drive may comprise a spring.

The insertion device may also include a trigger-type actuator to control the release of the carriage. The trigger-type actuator may include a button and a trigger spring.

The carriage includes a pillar in the center. A pair of fingers may be attached on opposite sides of the pillar at a forward end thereof for releasably receiving and retaining the needle assembly. The carriage may also include a pair of trigger arms that project generally rearwardly from a platform that is adjacent to a rear end of the pillar. The trigger arms may include out-turned trigger fingers - i.e., extending laterally away from a centerline of the trigger arm - at a rear or distal end of the trigger arm. The carriage includes at least one barb and, in some instances, a pair of barbs that project generally forwardly from the platform and include edges at the front or distal end of the platform to limit the slide distance of the carriage and prevent it from disengaging from the housing. The carriage also comprises a frame at the front end of the pillar to accommodate a main body of the needle assembly.

Optionally, the housing includes one or more guide slots on an inner wall configured to guide the carriage. A bottom end of the housing may be larger in diameter or width than a diameter or width of a top end of the housing.

The trigger-type actuator may include a button and a trigger spring. The button optional comprises a cylindrical sleeve with a tapered or ramped leading-edge face configured to engage a ramped outer face or faces of the trigger fingers.

A lower end of the housing may include a curvature to adapt to the skin, the hands, or the fingers of a user or patient. Two inward curving outlines may be formed at the lower end of the housing. Further, the insertion device may also include a curvature to rest a user or patient's thumb and middle finger and configured to press against a patient's skin when setting the inserter on the patient's skin. The inserter may include haptic or sensitization points to distract the brain from the needle puncturing the patient's skin.

The foregoing has outlined rather broadly the features of the present disclosure in order that the detailed description that follows may be better understood. Additional features and advantages of the disclosure will be described hereinafter, which form the subject of the claims.

### BRIEF DESCRIPTION OF THE DRAWINGS

In order that the manner in which the above-recited and other enhancements and objects of the disclosure are obtained, a more particular description of the disclosure briefly described above will be rendered by reference to specific embodiments thereof which are illustrated in the appended drawings. Understanding that these drawings depict only typical embodiments of the disclosure and are therefore not to be considered limiting of its scope, the disclosure will be described with additional specificity and detail through the use of the accompanying drawings in which:
Fig. 1 shows a needle assembly which can be used in the present invention;
Fig. 2 is an exploded view of an insertion device, according to one embodiment of the present invention;
Fig. 3 is a perspective view of an insertion device;
Fig. 4 is a cross-sectional view of an insertion device with a carriage in the distal position;
Fig. 5 is a cross-sectional view of an insertion device with a carriage in the proximal position;
Fig. 6 is a bottom view of an insertion device in Fig. 3.

### DETAILED DESCRIPTION

The particulars shown herein are by way of example and for purposes of illustrative discussion of the preferred embodiments of the present disclosure only and are presented in the cause of providing what is believed to be the most useful and readily understood description of the principles and conceptual aspects of various embodiments of the disclosure. In this regard, no attempt is made to show structural details of the disclosure in more detail than is necessary for the fundamental understanding of the disclosure, the description taken with the drawings making apparent to those skilled in the art how the several forms of the disclosure may be embodied in practice.

The following definitions and explanations are meant and intended to be controlling in any future construction unless clearly and unambiguously modified in the following examples or when application of the meaning renders any construction meaningless or essentially meaningless. In cases where the construction of the term would render it meaningless or essentially meaningless, the definition should be taken from Webster's Dictionary 3^{rd} Edition.

As used herein to describe the insertion device, or any of the relative positions of the components of the present invention, the terms "axial" or "axially" refer generally to a longitudinal axis around which the components of the insertion device are preferably positioned, although not necessarily symmetrically there-around. The term "radial" refers generally to a direction normal to the longitudinal axis. The terms "proximal," "rearward," or "backward" refer generally to an axial direction in the direction away from the needle. The teams "distal," "front," "frontward," "" or "forward" refer generally to an axial direction in the direction close to the needle. 5

As shown in the exemplary drawings, an insertion device is provided for quick and easy transcutaneous placement of a medical needle, particularly such as an insertion needle assembly 600 as depicted in Fig. 1, although the insertion device may be used with any type of needle. The insertion device includes a trigger-type actuator mechanism for transcutaneous placement of the needle assembly 600 with a controlled speed and force.

The insertion device of the present invention represents a simple device which can be used by the patient to quickly and easily place the needle assembly 600 at a selected medication insertion site. The insertion device is designed to project the infusion set toward the patient's skin at a controlled force and speed for quickly piercing the skin in a manner ensuring proper placement of the needle assembly 600, while minimizing patient anxiety and/or discomfort. Improper and/or partial placement of the insertion needle is thus avoided.

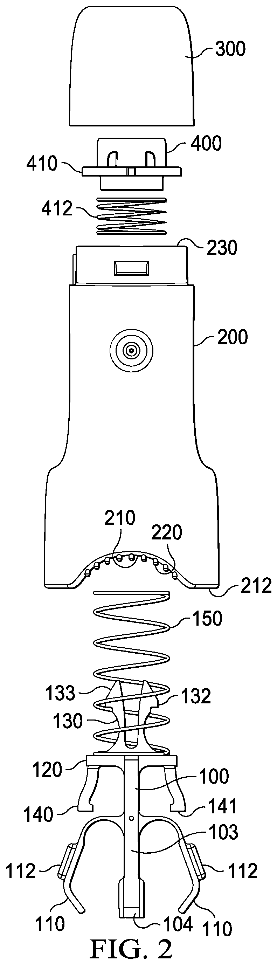

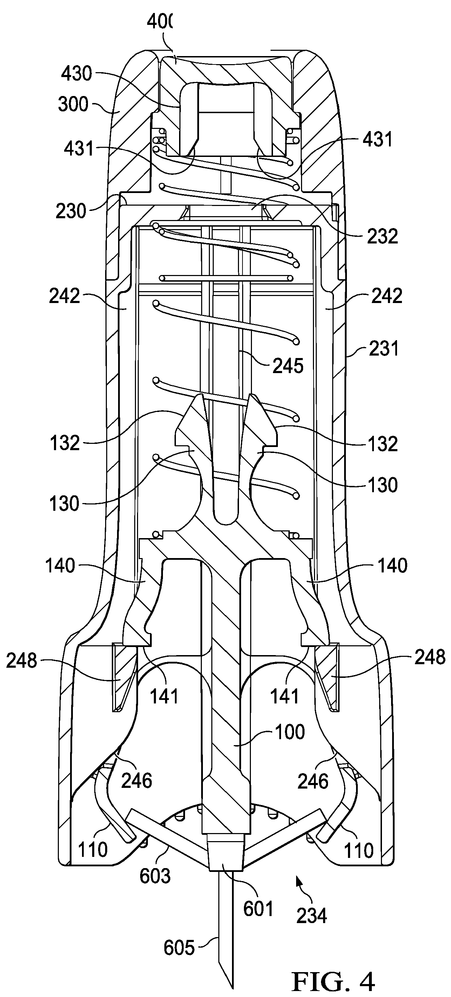

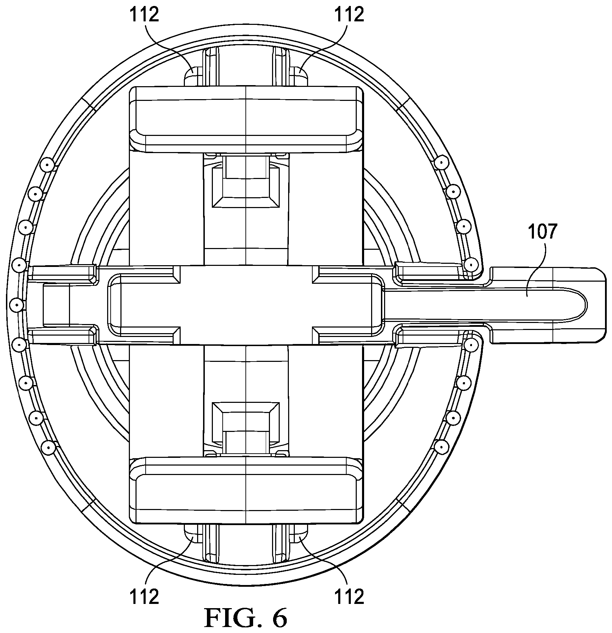

As shown in an embodiment in Figs. 2-6, the insertion device comprises a housing 200 (Fig. 2), which optionally may be cylindrical or any other shape. The housing 200 comprises a shell 231 with a distal opening 234 and a proximal opening 232 (Fig. 4); the housing 200 may be made of any of a number of materials including plastics, metals, and glass. In an embodiment, the housing 200 is made of plastic.

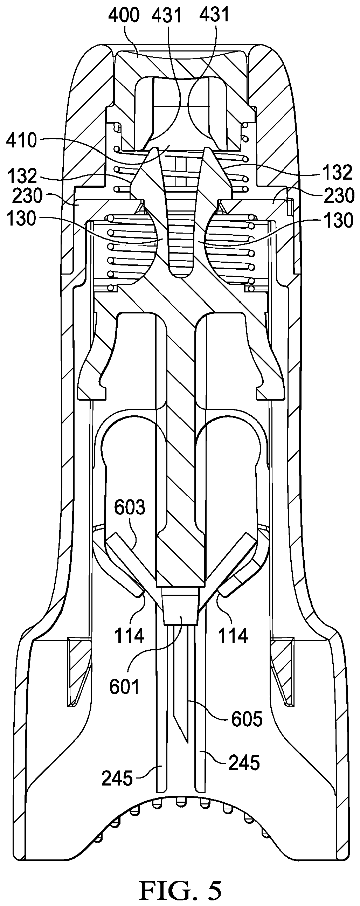

An interior space defined by the shell 231 can receive a carriage 100 such that the carriage 100 can longitudinally slide within the shell 231 between a distal position (Fig. 4) and a proximal position (Fig. 5).

The carriage 100 may include a pillar 103 in or near a center of the carriage 100. The carriage also may include at least one and, in some examples, a pair of fingers or a plurality of fingers 110 attached to and extending laterally away from the pillar 103. The fingers 110 may be positioned on opposite sides of the pillar 103, substantially equidistantly around the pillar (within +/- 10 degrees of equidistant), or radially around the pillar 103, such as at 180 degrees, 120 degrees, 90 degrees, 60 degrees and so forth depending on the number of fingers 103 that are present. The fingers 103 may be positioned at a forward or distal end of the carriage 100 and configured to releasably receive and retain the insertion needle assembly 600. The fingers 110 may be made of a flexible material (including plastic and thin metal) such that they can expand and draw back radially away from and towards the pillar 103 in a manner to be described in more detail herein.

The carriage 100 may also include at least one and, in some examples, two or more trigger fingers 132 at a rear or proximal end of the carriage 100 spaced apart from the front or the distal end of the carriage 100. The trigger fingers 132 may be configured to cooperate with the tapered or ramped leading-edge face of the trigger actuator or button 400 (Figs. 4 and 5) that may be mounted on a rear or proximal end of the housing 200. The trigger fingers 132 interact with the shoulder 230 of the proximal opening 232 to hold the carriage 100 in a retracted position (Fig. 5) in which the needle 605 is sheathed within the housing 200 against the force of a compressed drive spring 150. A trigger button 400 of the actuator assembly is configured to or adapted for fingertip depression to compress the trigger fingers 132 so as to release them from the shoulder 230 of the proximal opening 232 and thereby release the carriage 100 for spring-loaded travel toward the forward or distal position and corresponding transcutaneous placement of the insertion needle assembly 600 through the patient's skin.

The carriage 100 additionally may include at least one and, in some examples, a pair or a plurality (which encompasses a pair or more) of trigger arms 130 which project generally rearwardly from a platform 120 that is adjacent to a rear or proximal end of the pillar 103. The trigger arms 130 may include trigger fingers 132 that include out-turned, i.e., structures that extend laterally away from a centerline of the carriage 100 proximate a rear or proximal end of the trigger fingers 132. The trigger fingers 132 may include a proximal end, a ramp portion that extends distally and laterally outwardly from the proximal end, and a step or tooth at a distal end of the ramp portion. The trigger fingers 132 are adapted and sized for partial radial compression toward each other and the centerline of the carriage 100 as the trigger fingers 132 pass through the proximal opening 232 of cylindrical housing 200 when the carriage 100 is displaced from the distal position to the retracted position. As the retracted position is reached, the trigger fingers 132 are spring-loaded by the resiliency of the trigger arms 130 to move outwardly to be hooked by an outer face of a shoulder 230 of the proximal opening 232 of the cylindrical housing 200. In this position, as shown in Fig. 5, the triggers fingers 132 retain the carriage 100 in the retracted position.

A drive spring 150 may be mounted within the cylindrical housing 200 to react between the trigger-type actuator assembly 410 and the carriage 100. The drive spring 150 may be a coil spring positioned between the platform 120 and an inner face of the shoulder 230 of the proximal opening 232 of the cylindrical housing 200. The drive spring 150 optionally may partially or fully encompass and/or encircle the trigger arms 130. The drive spring 150 (Fig 2) normally biases the carriage 100 toward the distal or extended position.

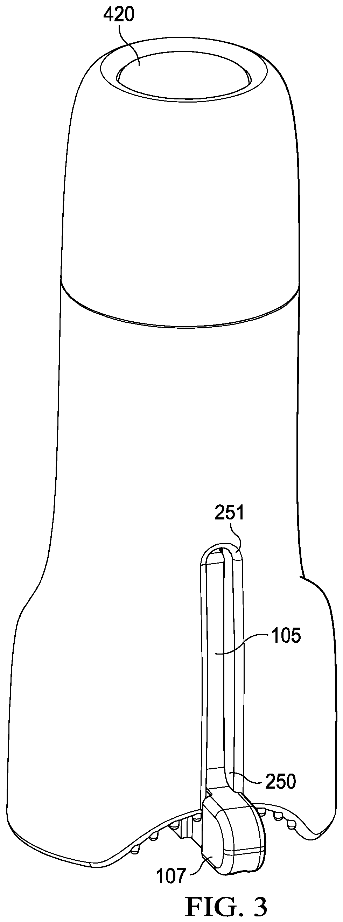

The carriage 100 optionally includes a pushing handle 107 (Fig. 3) attached on the pillar 103 can be pressed rearwardly or proximally along the housing 200 to move the carriage 100 from the extended position in Fig. 4 to the retracted position in Fig.5. In the process of retracting the needle 605 and the carriage 100 with the pushing handle 107, the trigger fingers 132 pass through a proximal opening 232 of the cylindrical housing 200 to engaging a shoulder 230 of the proximal opening 232. In this regard, the trigger fingers 132 have ramped outboard faces 133 (Fig. 2) to accommodate movement of the trigger fingers 132 radially toward each other as they pass through the proximal opening 232. When the trigger fingers 132 pass entirely through the proximal opening 232, the spring resilience of the trigger arms 130 is sufficient to spread the trigger fingers 132 so that they engage the shoulder 230. In this retracted carriage position, the drive spring 150 is retained in a compressed condition with the needle assembly 600 withdrawn into the interior of the housing 200 in a spaced relation to the patient's skin.

The trigger-type actuator assembly 410 optionally comprises a button 400, a shoulder (unlabeled, but generally indicated by the lead line for trigger-type actuator assembly 410), and a trigger spring 412 (Fig. 2). The button 400 and the trigger spring 412 may be positioned within or accommodated in a cap 300 at an upper or proximal end of the cylindrical housing 200. The shoulder may interact with the cap 300 so as to retain the button 400 within the cap against the urging of the trigger spring 412. The cap 300 and the cylindrical housing 200 may be coupled together through various manners. In various embodiments, the cap 300 and cylindrical housing 200 may be engaged through threads, interference fit, snap fit, or as in Fig. 2 the cap 300 and the cylindrical housing 200 may be engaged by grooves or recesses within an outer surface of the housing 200 and complementary projections in an inners surface of the cap 300 or vice-versa.

The button 400 may comprise a cylindrical sleeve 430 configured to slide within the cap 300 longitudinally. The cylindrical sleeve 430 may include a tapered or ramped leading-edge face 431 for engaging the ramped outboard faces 133 of the trigger fingers 132. The tapered leading-edge face 431 interact with the ramped outboard faces of the trigger fingers 132 to radially compress the trigger arms 130 and release the carriage 100 for spring-loaded travel from the retracted position (Fig. 5) to the extended or distal position (Fig. 4). The button 400 is exposed for fingertip depression at the top of the cap 300 to move the cylindrical sleeve 430 into releasing engagement with the trigger fingers 132.

The trigger spring 412 is mounted within the cap 300 and may comprise a coil spring positioned between an upper face of the shoulder 230 and the button 400 (Fig. 2). The drive spring 150 normally biases the button 400 towards the proximal position. However, a fingertip can depress the button 400 at the top of the cap 300 to move the cylindrical sleeve 430 against the trigger fingers 132 and release the carriage 100 from the retracted position (Fig. 5) to the extended position (Fig. 4).

Optionally, the button 400 may be a recessed button with a top surface 420 being lower than a top surface of the cap 300 to prevent accidental release.

The carriage 100 may further include at least one and, in some examples, at least a pair or a plurality (encompassing a pair or more) of barbs 140 that project generally proximately from the platform 120. The barbs 140 may include edges 141 at the front or distal ends of the barbs 140. These barbs 140 are adapted and sized to fit in the housing 200 with the edges 141 configured to slidably engage slots 242 (Fig. 4) on an inner wall of the housing 200 as the barbs 140 move rearward when the carriage 100 is displaced from the extended position to the retracted position. When the button 400 is depressed to release the carriage 100, the compressed drive spring 150 drives the carriage 100 distally to the extended position (Fig. 4) and the barbs 140 move with the carriage 100 until the edges 141 are blocked by the bosses 248 on the inner shell of the housing 200. The bosses 248 limit the distance of the carriage 100 and prevent it from extending too far from or completely out of the housing 200. In the reverse as the pushing handle 107 is actuated to retract the carriage 100, the trigger fingers 132 are spring-loaded by the resiliency of the trigger arms 130 to move outwardly to be hooked by outer face of the shoulder 230 of the proximal opening 232 of cylindrical housing 200. In this position, as shown in FIG. 5, the triggers fingers 132 retain the carriage 100 in the retracted position.

In order to limit the sliding of the carriage 100 in the housing 200, the housing 200 may include guide slots 245 on the inner wall of the housing 200. The guide slots 245 may be parallel to the longitudinal axis of the housing 200. Optionally, the pillar 103 may have a rectangular shape, and the two sidewalls 105 (Fig. 3), which optionally may not connect to the fingers 110, fit in two of the guide slots 245 respectively. Each of the fingers 110 may include a convex shape and a through groove 112 configured to engage a guide wedge 246 on the inner wall of the housing 200. The pair of barbs 140 are also limited by a pair of guide slots 242, the bosses 248 are located in the guide slots 242, limiting the potential travel and movement of the pair of barbs 140. In other words, these various structures act to constrain the movement of the carriage 100 and, by extension, the needle 605 in a controlled and predictable manner.

Optionally, a bottom or distal end of the housing 200 is larger in width or diameter than a width or diameter of a top or proximal end of the housing 200. The bottom end of the housing 200 is big enough to accommodate the fingers 110 when the carriage 100 is in the proximal or retracted position. The pushing handle 107, as discussed, may be attached on the side of pillar 103, and the pushing handle 107 may extend through a through groove 250 (Fig. 3) in the housing 200. The through groove 250 may be provided along one of the guide slots 245 to allow the pushing handle 107 to press rearwardly against the carriage 100 to move the carriage to the retracted position (Fig. 4). Optionally, the through groove 250 may include a rear edge 251 proximate a rear or proximal end of the through groove 250 to limit the backward distance of travel of the pushing handle 107 and, in turn, the carriage 100.

In an embodiment, a frame 104 is formed at the front end of the pillar 103 to accommodate the main body 601 of the needle assembly 600. Optionally, the frame 104 is a recess within the pillar 103 configured to receive the main body 601 of the needle assembly 600 therein. Upon using the insertion device, the carriage 100 is in the proximal position. The main body 601 of the needle assembly 600 is in the frame 104and the two wings 603 are arranged at the intervals between the fingers 110 and the pillar 103 respectively (Fig. 4); The pushing handle 107 is moved rearwardly - to move the carriage 100 to the retracted position. The fingers 110 move backward, the fingertips 114 of fingers 110 engage the guide wedge 246 on the inner wall of the housing 200, radially compressing the fingers 110 towards the pillar 103 because of the reduced space of the housing 200. The two wings 603 are gripped between the fingers 110 and the pillar 103 respectively, such that the needle assembly 600 is on the locked position (Fig. 5).

The lower or distal end of the housing 200 has a curvature shaped to comfortable adapt the housing 200 to a patient's skin. For example, two inward curving outlines 210 may be formed at the lower end of the housing 200. Further, the housing 200 may include a curvature to rest a patient or user's thumb and middle finger to press against skin when setting the inserter on the patient's skin. The housing 200 may including haptic or sensitization points 220 to distract the patient from the actual needle puncture.

After the needle assembly 600 is arrange in the locked or retracted position (Fig. 5), the user can set the inserter on the patient's skin using one hand, with the housing 200 oriented generally perpendicular to the skin. The user then presses the button 400 to release the carriage 100 from the retracted position to the extended position (Fig. 4). The needle assembly 600 rapidly travels with carriage 100 with a controlled speed and force of insertion to ensure penetration of the patient's skin with minimal discomfort. The fingers 110 radially expand by resiliency to release the wings 603 of the needle assembly 600 because of the increased space for the fingers 110 to expand when the wings 603 move frontward. The inserter may then be reused.

All of the compositions and methods disclosed and claimed herein can be made and executed without undue experimentation in light of the present disclosure. While the compositions and methods of this disclosure have been described in terms of preferred embodiments, it will be apparent to those of skill in the art that variations may be applied to the compositions and methods and in the steps or in the sequence of steps of the methods described herein without departing from the concept, spirit and scope of the disclosure.

## Claims

1. An insertion device for receiving and moving a medical needle from a retracted position in which the medical needle is housed therein to an extended position in which the medical needle is exposed to a skin of a patient, the insertion device comprising:
a housing (200) including a shell (231) with a distal opening (234) and a proximal opening (232) spaced apart from the distal opening (234);
a carriage (100) configured to be slidably received within the shell (231) between an extended position and a retracted position;
wherein the carriage (100) includes elements to secure a needle assembly;
wherein the carriage (100) comprises a platform (120); and a pillar (103) coupled to the platform (120) and extending away from the platform (120) towards the distal opening (234),
wherein the pillar (103) comprises a frame configured to receive the medical needle and a plurality of fingers (110) coupled to the pillar (103) and extending laterally away from the pillar (103) and being spaced substantially equidistantly around the pillar (103), wherein the plurality of fingers (110) is configured to releasably receive and retain the medical needle;
wherein the carriage (100) comprises at least one trigger arm (130) coupled to the platform (120) and extending away from the platform (120) towards the proximal opening (232); and
wherein the carriage (100) comprises at least one barb (140) coupled to the platform (120) and extending away from the platform (120) towards the distal opening (234).

2. The insertion device of claim 1, comprising a drive configured to bias the carriage (100) relative to the housing (200).

3. The insertion device of claim 2, wherein the drive comprises a spring (150).

4. The insertion device of claim 1, wherein the at least one trigger arm (130) comprises a plurality of trigger arms (130); or
wherein the at least one trigger arm (130) includes a trigger finger (132) spaced proximally from the platform (120), optionally wherein the at least one trigger finger (132) includes a ramped face oriented towards the shell (231).

5. The insertion device of claim 1, wherein the at least one barb (140) comprises a plurality of barbs (140); or wherein the at least one barb (140) includes a front end spaced apart from the platform (120), the front end including an edge configured to interact with a boss (248) positioned within the shell (231).

6. The insertion device of claim 1, wherein the housing (200) comprising at least one guide slot (245) on an inner wall of the shell (231), wherein the at least one guide slot (245) is configured to receive partially therein the plurality of fingers (110).

7. The insertion device of claim 1 comprising a trigger-type actuator (410), wherein the trigger-type actuator (410) comprises: a cap (300) coupled to the housing (200); a button (400) positioned within the housing (200); and, a trigger spring (412) positioned between the button (400) and a shoulder of the housing (200), the shoulder proximate the proximal opening (232) of the housing (200), wherein the trigger spring (412) is configured to urge the button (400) towards a proximal end of the cap (300).

8. The insertion device of claim 7, wherein the at least one trigger arm (130) includes a trigger finger (132) spaced proximally from the platform (120), and wherein
the at least one trigger finger (132) includes a ramped face (133) oriented towards the shell (231); and
wherein the button (400) comprises a sleeve (430) including a tapered face (431) oriented away from the shell (231), the tapered face (431) configured to interact with the ramped face (133) of the at least one trigger finger (132) so as to cause the at least one trigger finger (132) to flex inward away from the shell (231) when the button (400) is depressed, thereby releasing the carriage (100).

## Patentansprüche

1. Einsetzvorrichtung zum Aufnehmen und Bewegen einer medizinischen Nadel aus einer zurückgezogenen Position, in der die medizinische Nadel darin untergebracht ist, in eine ausgefahrene Position, in der die medizinische Nadel mit der Haut eines Patienten in Kontakt ist, wobei die Einsetzvorrichtung umfasst:
ein Gehäuse (200), das eine Außenhülle (231) mit einer distalen Öffnung (234) und einer proximalen Öffnung (232), die von der distalen Öffnung (234) beabstandet ist, einschließt;
einen Schlitten (100), der so konfiguriert ist, dass er innerhalb der Außenhülle (231) zwischen einer ausgefahrenen Position und einer zurückgezogenen Position verschiebbar aufgenommen ist;
wobei der Schlitten (100) Elemente zum Sichern einer Nadelanordnung einschließt;
wobei der Schlitten (100) eine Plattform (120); und eine Säule (103), die mit der Plattform (120) gekoppelt ist und sich von der Plattform (120) weg zu der distalen Öffnung (234) hin erstreckt, umfasst,
wobei die Säule (103) einen Rahmen umfasst, der so konfiguriert ist, dass er die medizinische Nadel und eine Vielzahl von Fingern (110) aufnimmt, die mit der Säule (103) gekoppelt sind und sich seitlich von der Säule (103) weg erstrecken und im Wesentlichen äquidistant um die Säule (103) beabstandet sind, wobei die Vielzahl von Fingern (110) so konfiguriert ist, dass sie lösbar die medizinische Nadel aufnehmen und zurückhalten;
wobei der Schlitten (100) mindestens einen Auslösearm (130) umfasst, der mit der Plattform (120) gekoppelt ist und sich von der Plattform (120) weg zu der proximalen Öffnung (232) hin erstreckt; und
wobei der Schlitten (100) mindestens eine Spitze (140) umfasst, die mit der Plattform (120) gekoppelt ist und sich von der Plattform (120) weg zu der distalen Öffnung (234) hin erstreckt.

2. Einsetzvorrichtung nach Anspruch 1, umfassend einen Antrieb, der so konfiguriert ist, dass er den Schlitten (100) in Bezug auf das Gehäuse (200) vorspannt.

3. Einsetzvorrichtung nach Anspruch 2, wobei der Antrieb eine Feder (150) umfasst.

4. Einsetzvorrichtung nach Anspruch 1, wobei der mindestens eine Auslösearm (130) eine Vielzahl von Auslösearmen (130) umfasst; oder
wobei der mindestens eine Auslösearm (130) einen Auslösefinger (132) einschließt, der proximal von der Plattform (120) beabstandet ist, wobei der mindestens eine Auslösefinger (132) optional eine abgeschrägte Seite einschließt, die zu der Außenhülle (231) hin ausgerichtet ist.

5. Einsetzvorrichtung nach Anspruch 1, wobei die mindestens eine Spitze (140) eine Vielzahl von Spitzen (140) umfasst; oder wobei die mindestens eine Spitze (140) ein Vorderende, das von der Plattform (120) beabstandet ist, einschließt, wobei das Vorderende eine Kante einschließt, die so konfiguriert ist, dass sie mit einem Ansatz (248) interagiert, der innerhalb der Außenhülle (231) positioniert ist.

6. Einsetzvorrichtung nach Anspruch 1, wobei das Gehäuse (200) mindestens einen Führungsschlitz (245) an einer Innenwand der Außenhülle (231) umfasst, wobei der mindestens eine Führungsschlitz (245) so konfiguriert ist, dass er darin die Vielzahl von Fingern (110) teilweise aufnimmt.

7. Einsetzvorrichtung nach Anspruch 1, umfassend einer Auslöse-(410), wobei die Auslöse-Betätigungsvorrichtung (410) umfasst: eine Kappe (300), die mit dem Gehäuse (200) gekoppelt ist; einen Knopf (400), der innerhalb des Gehäuses (200) positioniert ist; und eine Auslösefeder (412), die zwischen dem Knopf (400) und einer Schulter des Gehäuses (200) positioniert ist, wobei die Schulter in der Nähe der proximalen Öffnung (232) des Gehäuse (200) gelegen ist, wobei die Auslösefeder (412) so konfiguriert ist, dass sie den Knopf (400) zu einem proximalen Ende der Kappe (300) drängt.

8. Einsetzvorrichtung nach Anspruch 7, wobei der mindestens eine Auslösearm (130) einen Auslösefinger (132) einschließt, der proximal von der Plattform (120) beabstandet ist, und wobei der mindestens eine Auslösefinger (132) eine abgeschrägte Seite (133) einschließt, die zu der Außenhülle (231) hin ausgerichtet ist; und
wobei der Knopf (400) eine Muffe (430) umfasst, die eine verjüngte Seite (431) einschließt, die von der Außenhülle (231) weg ausgerichtet ist, wobei die verjüngte Seite (431) so konfiguriert ist, dass sie mit der abgeschrägten Seite (133) des mindestens einen Auslösefingers (132) so interagiert, dass sie bewirkt, dass sich der mindestens eine Auslösefinger (132) von der Außenhülle (231) weg nach innen biegt, wenn der Knopf (400) heruntergedrückt wird und dadurch der Schlitten (100) freigegeben wird.

## Revendications

1. Dispositif d'insertion pour recevoir et déplacer une aiguille médicale depuis une position rétractée, dans laquelle l'aiguille médicale est logée à l'intérieur de celui-ci, jusqu'à une position étendue, dans laquelle l'aiguille médicale est exposée à la peau d'un patient, le dispositif d'insertion comprenant :
un boîtier (200) incluant une coque (231) présentant une ouverture distale (234) et une ouverture proximale (232) espacée de l'ouverture distale (234) ;
un chariot (100), configuré pour être reçu de manière coulissante à l'intérieur de la coque (231) entre une position étendue et une position rétractée ;
dans lequel le chariot (100) inclut des éléments pour fixer un ensemble aiguille ;
dans lequel le chariot (100) comprend une plateforme (120) ; et un montant (103) couplé à la plateforme (120) et s'étendant à partir de la plateforme (120) vers l'ouverture distale (234),
dans lequel le montant (103) comprend un cadre configuré pour recevoir l'aiguille médicale et une pluralité de doigts (110) couplés au montant (103) et s'étendant latéralement à partir du montant (103) et étant espacés de manière sensiblement équidistante autour du montant (103), dans lequel la pluralité de doigts (110) est configurée pour recevoir et retenir de manière amovible l'aiguille médicale ;
dans lequel le chariot (100) comprend au moins un bras de déclenchement (130) couplé à la plateforme (120) et s'étendant à partir de la plateforme (120) vers l'ouverture proximale (232) ; et
dans lequel le chariot (100) comprend au moins un ergot (140) couplé à la plateforme (120) et s'étendant à partir de la plateforme (120) vers l'ouverture distale (234).

2. Dispositif d'insertion selon la revendication 1, comprenant un entraînement configuré pour solliciter le chariot (100) par rapport au boîtier (200).

3. Dispositif d'insertion selon la revendication 2, dans lequel l'entraînement comprend un ressort (150).

4. Dispositif d'insertion selon la revendication 1, dans lequel au moins un bras de déclenchement (130) comprend une pluralité de bras de déclenchement (130) ; ou dans lequel ledit au moins un bras de déclenchement (130) inclut un doigt de déclenchement (132) espacé de manière proximale de la plateforme (120), facultativement dans lequel ledit au moins un doigt de déclenchement (132) inclut une face en rampe orientée vers la coque (231).

5. Dispositif d'insertion selon la revendication 1, dans lequel ledit au moins un ergot (140) comprend une pluralité d'ergots (140) ; ou dans lequel ledit au moins un ergot (140) inclut une extrémité avant espacée de la plateforme (120), l'extrémité avant incluant un bord configuré pour interagir avec un bossage (248) positionné à l'intérieur de la coque (231).

6. Dispositif d'insertion selon la revendication 1, dans lequel le boîtier (200) comprend au moins une fente de guidage (245) sur une paroi interne de la coque (231), dans lequel ladite au moins une fente de guidage (245) est configurée pour recevoir partiellement en son sein la pluralité de doigts (110).

7. Dispositif d'insertion selon la revendication 1 comprenant un actionneur de type gâchette (410), dans lequel l'actionneur de type gâchette (410) comprend : un capuchon (300), couplé au boîtier (200) ; un bouton (400), positionné à l'intérieur du boîtier (200) ;
et un ressort de gâchette (412), positionné entre le bouton (400) et un épaulement du boîtier (200), l'épaulement étant au voisinage de l'ouverture proximale (232) du boîtier (200), dans lequel le ressort de gâchette (412) est configuré pour pousser le bouton (400) vers une extrémité proximale du capuchon (300).

8. Dispositif d'insertion selon la revendication 7, dans lequel au moins ledit un bras de déclenchement (130) inclut un doigt de déclenchement (132) espacé proximale de la plateforme (120), et dans lequel ledit au moins un doigt de déclenchement (132) inclut une face en rampe (133) orientée vers la coque (231) ; et dans lequel le bouton (400) comprend un manchon (430) incluant une face conique (431) orientée à l'opposé de la coque (231), la face conique (431) étant configurée pour interagir avec la face en rampe (133) dudit au moins un doigt de déclenchement (132) de manière à amener l'au moins un doigt de déclenchement (132) pour fléchir vers l'intérieur, à l'écart de la coque (231), lorsque le bouton (400) est enfoncé, libérant ainsi le chariot (100).
